# EUROPEAN PATENT SPECIFICATION

(11) **EP 0 971 889 B1**
(45) Date of publication and mention of the grant of the patent: **13.08.2003**
(21) Application number: 98902597.8
(22) Date of filing: 16.01.1998
(51) Int. Cl.: C07D 205/08, C07F 7/08, C07F 7/18

(54) **PROCESS FOR SYNTHESIZING CARBAPENEM INTERMEDIATES**
VERFAHREN ZUR HERSTELLUNG VON ZWISCHENPRODUKTEN DES CARBAPENEMS
PROCEDE DE SYNTHESE D'INTERMEDIAIRES DU CARBAPENEM

(30) Priority: 21.01.1997 US 36357 P; 21.03.1997 GB 9705855
(43) Date of publication of application: 19.01.2000
(73) Proprietor: MERCK & CO., INC., Rahway, New Jersey 07065 (US)
(72) Inventor: YASUDA, Nobuyoshi, Rahway, NJ 07065 (US); YANG, Chunhua, Rahway, NJ 07065 (US)
(74) Representative: Hiscock, Ian James
(86) International application number: US9800874
(87) International publication number: WO98031666

(56) References cited:
- EP-A- 0 336 143
- EP-A- 0 451 764
- US-A- 4 960 879
- US-A- 5 463 047
- SCHMITT S.M. ET AL.: "The synthesis of 2-(functionalized methyl)-1.beta.-methylcarbapenems" THE JOURNAL OF ANTIBIOTICS, JAPAN ANTIBIOTICS RESEARCH ASSOCIATION, TOKYO, JP, vol. 41, no. 6, June 1988 (1988-06), pages 780-787, XP000942903
- UEDA Y. & MAYNARD S.C.: "Highly regioselective formation of bromohydrins by reaction of epoxy-azetidinones with MgBr2: An alternative route to 4-bromomethylcarbonylmethyl-2-azetidinone, a key carbapenem precursor" TETRAHEDRON LETTERS, ELSEVIER SCIENCE PUBLISHERS, AMSTERDAM, NL, vol. 29, no. 41, 1988, pages 5197-5200, XP000942902

## Description

The present invention relates to the synthesis of carbapenem intermediates. The invention is particularly useful in that it facilitates the presence of a 1β-methyl group upon cyclization to form the non-beta lactam ring of the carbapenem nucleus.

The side chain which is attached at position two of the carbapenem nucleus can be introduced prior to non-beta lactam ring cyclization. This reduces the number of steps which are necessary to produce the final compound. The side chain can be in protected or unprotected form, or a precursor of the side chain can be used, such as a coupling moiety, which can be present in protected or unprotected form prior to cyclization. This facilitates the addition of the side chain. Thus, the process described herein has extended utility, in that many different carbapenem antibiotics can be synthesized.

These and other objects and advantages will be apparent from the teachings contained herein.

EP-A-0336143 (Shionogi Seiyaku Kabushiki Kaisha) discloses intermediates for synthesizing 1-β-alkyl-1-carbapenem. The intermediates are prepared stereoselectively by treating 4-(leaving group substituted) azetidin-2-one with a trans-2-(leaving group substituted)methyl-3-alkylacrylic acid and a reducing metal.

EP-A-0451764 discloses intermediates to carbapenems of formula II: 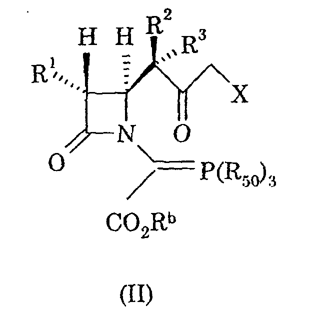 in which R^{b} is a carboxylic acid protecting group, R¹ is hydrogen, lower alkyl, lower alkoxy or hydroxyalkyl, R² and R³ are hydrogen, lower alkyl, lower alkoxy or lower alkylthio, X is halogen and R⁵⁰ is an aryl or alkyl group.

Such compounds can be made by reacting the corresponding methanol ketal at -20°C with hydrobromic acid. This compound, a 3-(1-hydroxyethyl)azetidinone, is made by reacting the corresponding 3-[1-[(2-trichloroethoxy)carbonyl]oxy]-ethylazetidinone with zinc dust in 90% aqueous acetic acid. This compound is prepared by the method of GB-A-2092147.

Schmitt et al, J. Antibiotics, XLI(6), 1988, 780-787 discloses compounds differing from those of formula I of the present invention by having a trialkylsilyl, hydrogen or alkyl ester moiety in place of the halogen atom.

Ueda and Maynard, Tet. Lett., 29(41), 1988, 5197-5200 discloses a compound (7) having substituents, corresponding to formula I below, R₁=P'=H, Hal=Br and P=SiMe₂t-Bu. It is produced by oxidation of the corresponding bromohydrin with pyridinium chlorochromate in CH₂Cl₂. This compound is made by reacting the corresponding epoxy-azetidinone with magnesium bromide in tetrahydrofuran at 0-5°C.

### SUMMARY OF THE INVENTION

A process of synthesizing a compound of formula 1 is described: 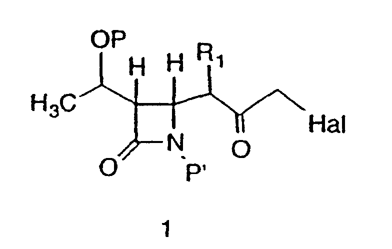 wherein P and P' independently represent H or a protecting group, R¹ represents H or C₁₋₄ alkyl, and
Hal represents a halogen selected from Cl, Br and I, comprising:
reacting a compound of formula 2: 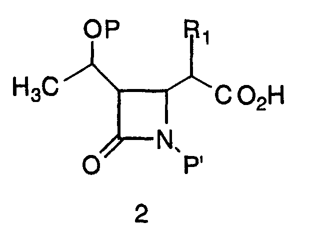 wherein P, P' and R₁ are as defined above with a di-C₁₋₄ alkyl hydroxylamine in the presence of a carbodiimide to produce a compound of formula 3: 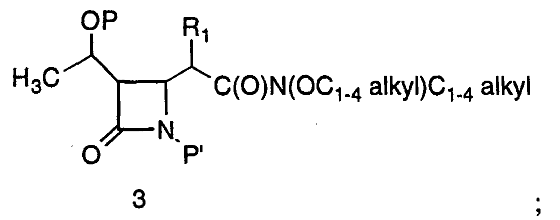
reacting compound 3 with a compound of formula 4:

   MetCH₂SiR₂R₃R₄ 4
wherein Met represents lithium or halomagnesium, and R₂, R₃ and R₄ are C₁₋₄ alkyl or C₁₋₄ alkoxy, and the halo portion of halomagnesium is Cl, Br or I,
to produce a compound of formula 5: 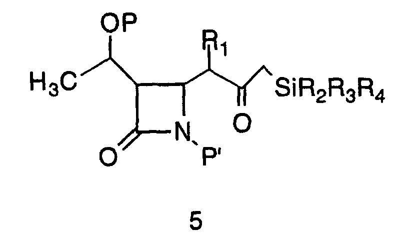 and reacting compound 5 with a halogenating agent to produce a compound of formula 1.

### DETAILED DESCRIPTION OF THE INVENTION

The invention is described herein in detail using the terms defined below unless otherwise specified.

Hal and halo mean Cl, Br and I selected on an independent basis.

Preferred alkyl groups include methyl, ethyl, n-propyl, isopropyl, n-butyl and t-butyl. More preferred alkyl groups are methyl, ethyl, isopropyl and t-butyl.

The term "carbodiimide" is used in the conventional sense and refers to compounds which contain the moiety: -NH=C=NH-. Examples include 1-(3-dimethylaminopropyl)-3-ethylcarbodiimide (EDC) and 1,3-dicyclohexylcarbodiimide (DCC).

The intermediate compounds synthesized in the present invention have asymmetric centers and occur as racemates, racemic mixtures, and as individual diastereomers. The processes of synthesizing all such isomers, including optical isomers, are included in the present invention.

In the invention described herein, a process of synthesizing a compound of formula 1 is conducted. 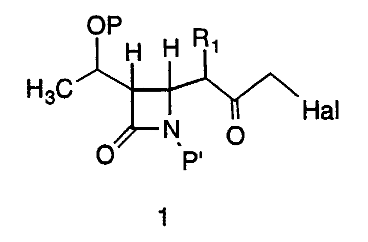 P and P' each independently represent H or a protecting group. R₁ represents H or C₁₋₄ alkyl, and Hal represents a halogen selected from Cl, Br and I.

A compound of formula 2: 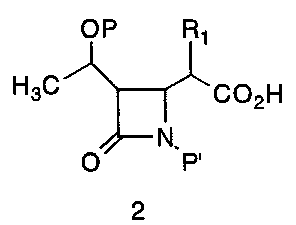 wherein P, P' and R₁ are as defined above is reacted with a di(C₁₋₄) alkyl hydroxylamine in the presence of a carbodiimide to produce a compound of formula 3: 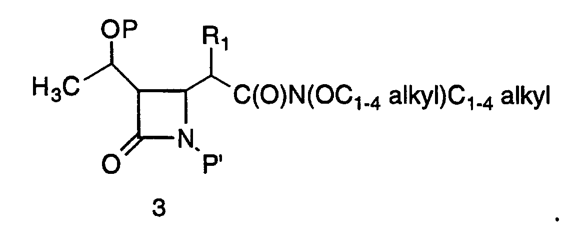 Compound 3 is reacted with a silyl compound of formula 4:

MetCH₂SiR₂R₃R₄ 4

in which Met represents lithium or halomagnesium. The halo portion of halomagnesium is Cl, Br or I. R₂, R₃ and R₄ are independently C₁₋₄ alkyl or C₁₋₄ alkoxy. This produces a compound of formula 5: 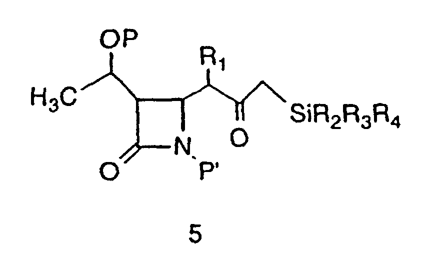 Compound 5 is reacted with a halogenating agent to produce a compound of formula 1.

Certain intermediate compounds are also disclosed of formula 1": 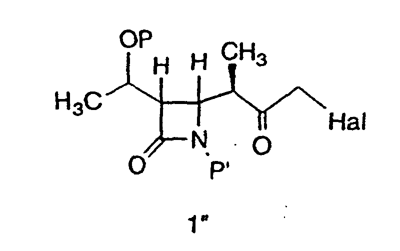 wherein P and P' each independently represent H or a protecting group and Hal represents a halogen selected from Cl, Br and I.

In another aspect of the invention, a compound represented by formula 3 or 5: 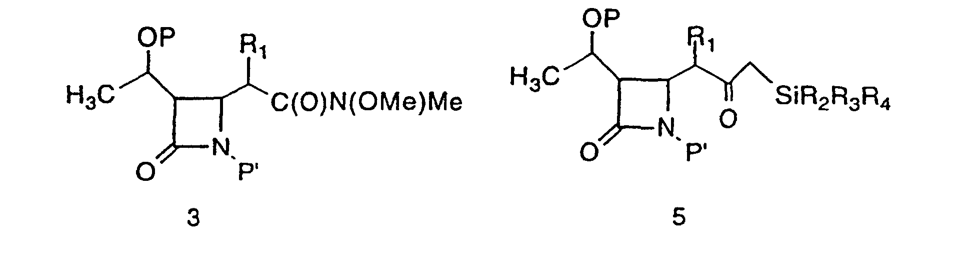 is included wherein:
R₁ represents H or C₁₋₄ alkyl,
P and P' independently represent H or a protecting group as defined below, and R₂, R₃ and R₄ are C₁₋₄ alkyl or C₁₋₄ alkoxy.

In a preferred aspect of the invention, P' represents H.

In another preferred aspect of the invention, R₁ represents C₁₋₄ alkyl, preferably, R₁ represents methyl, and most preferably methyl in the beta configuration.

In another preferred aspect of the invention, the carbodiimide is selected from EDC and DCC. Most preferably, the carbodiimide is EDC.

In another preferred aspect of the invention, zero or one of R₂, R₃ and R₄ represents C₁₋₄ alkoxy, and the others represent C₁₋₄ alkyl. More preferably, R₂, R₃ and R₄ represent methyl, or one of these variables represents methoxy or isopropyloxy, and the others represent methyl.

In another preferred aspect of the invention, the halo portion of halomagnesium is Cl.

In another preferred aspect of the invention, the halogenating agent is selected from the group consisting of Br₂, I₂, Cl₂, ICl , SO₂Cl₂ , N-chlorosuccinimide (NCS) and N-bromosuccinimide (NBS).

In a more preferred aspect of the invention, the halogenating agent is Br₂ , ICl or SO₂Cl₂.

A preferred process of synthesizing a compound of formula 1: 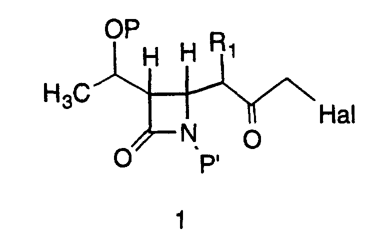 is included wherein P and P' each independently represent H or a protecting group, R¹ represents H or C₁₋₄ alkyl, and
Hal represents a halogen selected from Cl, Br and I, which comprises:
reacting a compound of formula 2: 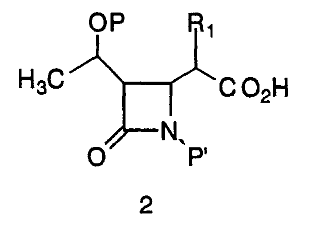 wherein P, P' and R₁ are as defined above with N, O-dimethylhydroxylamine in the presence of EDC or DCC to produce a compound of formula 3: 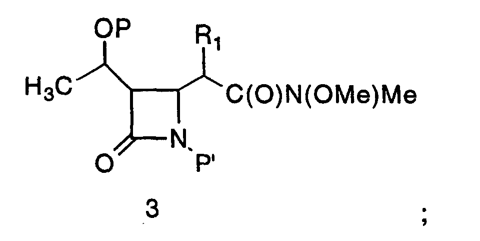
reacting compound 3 with a compound of formula 4:

   MetCH₂SiR₂R₃R₄ 4
wherein Met represents lithium or halomagnesium, and R₂, R₃ and R₄ are C₁₋₄ alkyl or C₁₋₄ alkoxy, and the halo portion of halomagnesium is Cl, Br or I,
to produce a compound of formula 5: 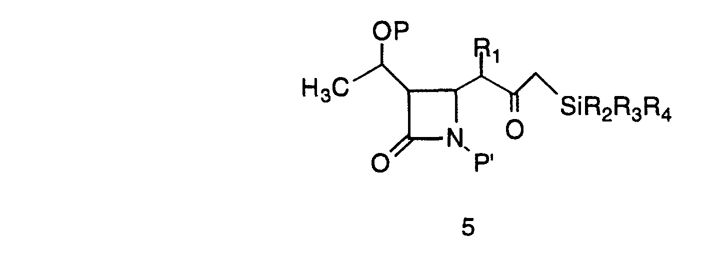 and reacting compound 5 with a halogen selected from Br₂, I₂ ,Cl₂ , NCS, NBS, ICl and SO₂Cl₂ to produce a compound of formula 1.

A more preferred process is set forth below. A compound of formula 1': 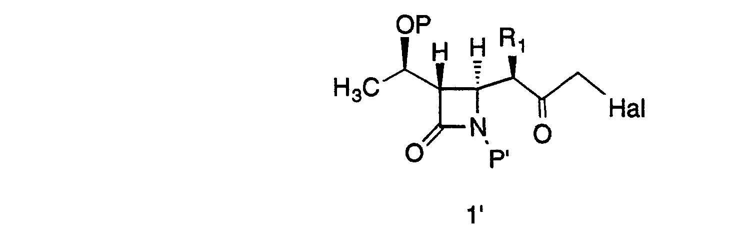 wherein P and P' each independently represent H or a protecting group, R¹ represents H or C₁₋₄ alkyl, and Hal represents a halogen selected from Cl, Br and I.
A compound of formula 2': 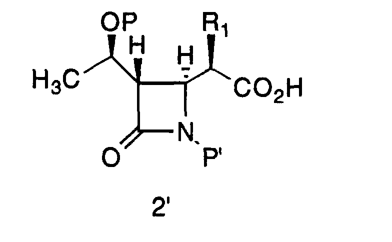 wherein P, P' and R₁ are as defined above is reacted with N,O-dimethylhydroxylamine in the presence of 1-(3-dimethylaminopropyl)-3-ethylcarbodiimide (EDC) or 1,3-dicyclohexylcarbodiimide (DCC) to produce a compound of formula 3': 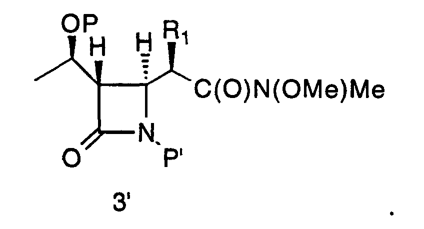 Compound 3' is reacted with a silyl compound of formula 4:

MetCH₂SiR₂R₃R₄ 4

Met represents lithium or halomagnesium, the halo portion of halomagnesium is Cl, Br or I, and R₂, R₃ and R₄ are independently C₁₋₄ alkyl or C₁₋₄ alkoxy. This produces a compound of formula 5': 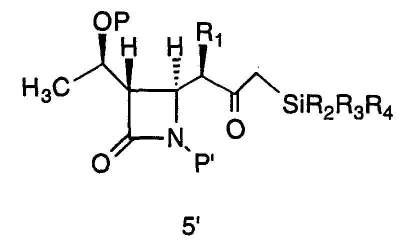 Compound 5' is reacted with a halogenating agent selected from Br₂, I₂ , Cl₂, NBS, NCS, ICI and SO₂Cl₂ to produce a compound of formula 1'.

The hydroxyl group at the 8-position of the carbapenem and the nitrogen in the beta lactam ring may be blocked until later in the synthesis, such as upon cyclization or when the final product is prepared. These blocking groups are readily removable, i.e., they can be removed, if desired, by procedures which will not cause cleavage or other disruption of the remaining portions of the molecule. Such procedures include chemical and enzymatic hydrolysis, treatment with chemical reducing or oxidizing agents under mild conditions, treatment with fluoride ion, treatment with a transition metal catalyst and a nucleophile, and catalytic hydrogenation.

Examples of suitable protecting groups are: t-butylmethylphenylsilyl, t-butyldiphenylsilyl, trimethylsilyl (TMS), triethylsilyl (TES), t-butyldimethylsilyl (TBDMS), o-nitrobenzyloxycarbonyl, p-nitrobenzyloxycarbonyl (PNZ), p-nitrobenzyl (PNB), benzyloxycarbonyl, t-butyloxycarbonyl, 2,2,2-trichloroethyloxycarbonyl and allyloxycarbonyl, Preferred hydroxyl protecting groups are TBDMS, TMS and TES.

Many other suitable protecting groups are known in the art. See, e.g., T.W. Greene, Protective Groups in Organic Synthesis, John Wiley & Sons, Inc., 1981 (Chapters 2 and 5).

Compound 1 is useful in the synthesis of carbapenem antibiotics. For example, compound 1 is useful in the synthesis of carbapenems such as those disclosed in EP Publication No. 0 451 764 A1 published on October 16, 1991 and incoroporated herein by reference, as well as other carbapenems.

The invention is illustrated in connection with the following examples.

### EXAMPLE 1

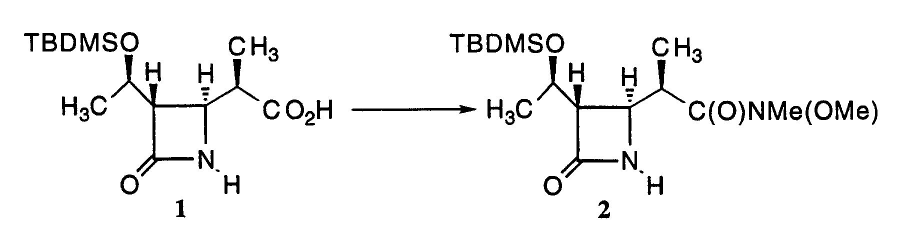

To a mixture of **1** (25.0 g), *N,O*-dimethylhydroxyamine hydrochloride (12.1 g), 1-hydroxybenzotriazole monohydrate (HOBT) (16.8 g), and 1-(3-dimethylaminopropyl)-3-ethylcarbodiimide hydrochloride (EDC) (23.8 g) in a mixture of water (150 mL) and methylene chloride (100 mL) was slowly added a solution of triethylamine (17.5 mL) in methylene chloride (50 mL) at ambient temperature. After stirring at ambient temperature overnight, additional *N*, *O*-dimethylhydroxyamine hydrochloride (1.6 g), 1-hydroxybenzotriazole monohydrate (2.7 g), triethylamine (2.4 mL), and 1-(3-dimethylaminopropyl)-3-ethylcarbodiimide hydrochloride (3.2 g) were added to the reaction mixture at ambient temperature. After confirming completion of the reaction by HPLC, the reaction mixture was diluted with a mixture of methylene chloride (45 mL) and saturated aqueous sodium hydrogen carbonate (45 mL).

The organic layer was separated, subsequently washed with 0.5 M phosphate buffer (140 mL) twice and saturated aqueous sodium chloride (140 mL), dried over magnesium sulfate, concentrated *in vacuo*. The residue was crystallized from ethyl acetate and hexanes to give crystalline **2** (28.0 g).

¹H NMR (250 MHz; CDCl₃): δ 5.98 (broad s, 1 H), 4.17 (m, 1 H), 3.84 (dd, *J* = 4.9 and 2.2 Hz, 1 H), 3.69 (s, 3 H), 3.18 (s, 3 H), 3.15 (m, 1 H), 2.98 - 2.95 (m , 1 H), 1.19 (d, *J* = 6.9 Hz, 3 H), 1.18 (d, *J* = 6.2 Hz, 3 H), 0.86 (s, 9 H), and 0.06 (s, 6 H).

¹³C NMR (62.9 MHz: CDCl₃): δ 175.2, 168.5, 65.2, 61.4, 61.3, 52.1, 37.7, 31.9, 25.7, 22.3, 17.8, 12.4, - 4.4, and - 5.1.

### EXAMPLE 2

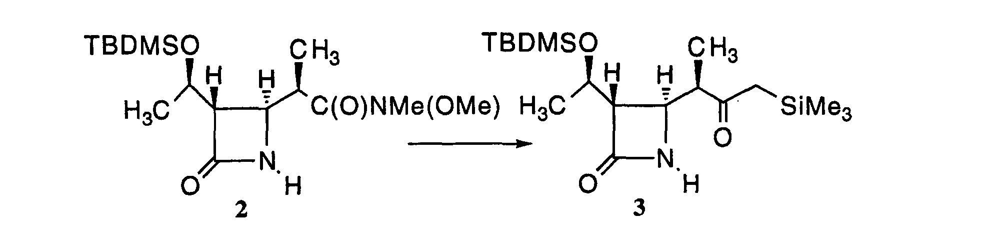

To a solution of **2** (3.4 g) in dry tetrahydrofuran (30 mL) was slowly added a solution of trimethylsilylmethyllithium (1 *M* hexanes; 35 mL) at -45 °C. After the completion of the addition, the mixture was allowed to be warmed up to -10 °C. The reaction was complete in 20 minutes at -10 °C. The reaction mixture was cooled down to - 40 °C and a solution of acetic acid (2 *M*) in dry tetrahydrofuran (17 mL) was added to the mixture at - 40 °C. The resulted mixture was diluted with dry tetrahydrofuran (8 mL) at - 40 °C. After stirred for 20 minutes at -40 °C, the reaction mixture was diluted with a mixture of diethyl ether (15 mL) and 0.5 *M* phosphate buffer (pH = 7.0; 15 mL). The organic layer was separated, washed with 0.5 *M* phosphate buffer (pH = 7.0; 15 mL), dried over magnesium sulfate, and concentrated *in vacuo* to give crude **3** (3.8 g). This compound was used for the next step without further purification.

Purified compound **3** (1.3 g) can be obtained by trituration of amide **3** (1.9 g) in hexanes (5 mL) at 0 °C.

¹H NMR (250 MHz; CDCl₃): δ 5.98 (broad s, 1 H); 4.16 (m, 1 H), 3.84 (m, 1 H), 2.85 (m, 1H), 2.71 (m, 1 H), 2.32 (d, *J* = 10.6 Hz, 1 H), 2.22 (d, *J* = 10.6 Hz, 1 H); 1.18 (d, *J* = 6.2 Hz, 3 H), 1.13 (d, *J* = 7.2 Hz, 3 H), 0.85 (s, 9 H), 0.118 (s, 3 H), 0.116 (s, 6 H), and 0.05 (s, 6 H).

¹³C NMR (62.9 MHz; CDCl₃): δ 211.5, 168.2, 65.5, 61.3, 51.1, 48.9, 37.8, 25.7, 22.5, 17.9, 11.1, -0.9, -4.3, and - 5.0.

### EXAMPLE 3

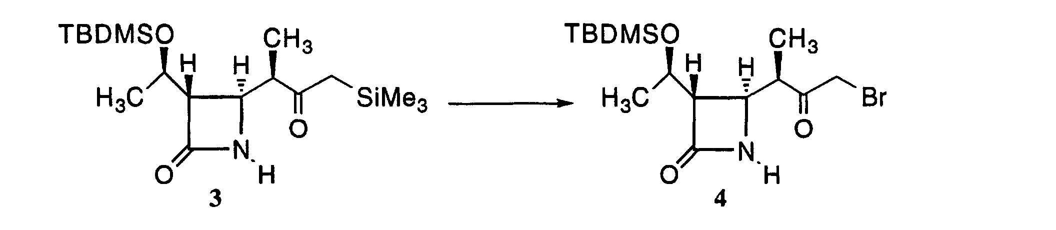

To a solution of **3** (3.76 g) in dry methylene chloride (35 mL) was slowly added a solution of bromine (2 *M*; 4.8 mL) in dry methylene chloride at - 35 °C. The reaction mixture was allowed to be warmed up to - 5 °C. The mixture was cooled down to -35 °C and additional the bromine solution (2 *M*; 1.2 mL) was added to the mixture. The mixture was stirred between - 30 and - 5 °C for 20 minutes. The reaction mixture was diluted with a mixture of methylene chloride (35 mL) and 0.5 *M* phosphate buffer (pH = 7.0; 35 mL). The organic layer was separated, subsequently washed with water (35 mL), 0.1 *M* sodium thiosulfate (35 mL) and water (35 mL), dried over magnesium sulfate, and concentrated *in vacuo* to give crude **4** (3.81 g). This compound could be used for the preparation of the biologically active carbapenems, without further purification. Analytically pure compound was obtained by silica gel column chromatography.

¹H NMR (250 MHz; CDCl₃): δ 5.87 (broad s, 1 H), 4.17 (m, 1 H), 3.99 (d, *J* = 12.5 Hz, 1 H), 3.92 (d, *J* = 12.5 Hz, 1 H), 3.90 (dd, *J* = 4.9 and 2.2 Hz, 1 H), 3.24 (m, 1 H), 2.94 (dd, J = 5.0 and 2.3 Hz, 1 H), 1.23 (d, *J* = 7.2 Hz, 3 H), 1.19 (d, *J* = 6.4 Hz, 3 H), 0.87 (s, 9 H), 0.07 (s, 3 H), and 0.06 (s, 3 H).

¹³C NMR (62.9 MHz; CDCl₃): δ 203.8, 168.3, 65.3, 61.8, 51.4, 45.4, 33.5, 25.7, 22.5, 17.9, 12.5, - 4.3, and - 5.0.

### EXAMPLE 4

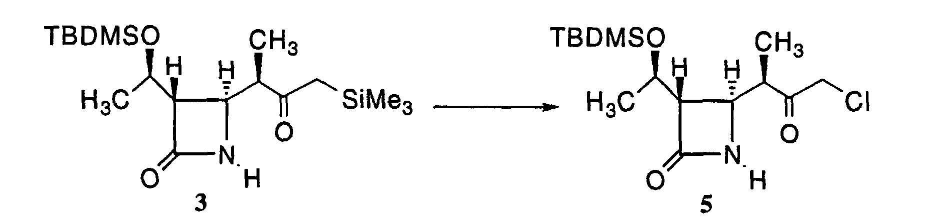

To a solution of **3** (1.88 g) in dry methylene chloride (15 mL) was slowly added a solution of sulfuryl chloride (2 *M*; 3.1 mL) in dry methylene chloride at - 30 °C. The mixture was stirred between - 30 and - 10 °C for 20 minutes. The reaction mixture was diluted with methylene chloride (15 mL) and 0.5 *M* phosphate buffer (15 mL). The organic layer was separated, subsequently washed with water (15 mL), 0.1 *M* sodium thiosulfate (15 mL) and water (15 mL), dried over magnesium sulfate, and concentrated *in vacuo* to give crude **5** (2 g). This compound was purified by trituration with cold hexanes (5 mL) to give pure **5** (1.3 g).

¹H NMR (250 MHz; CDCl₃): δ 5.89 (broad s, 1 H), 4.17 (m, 1 H), 4.15 (s, 2 H), 3.89 (dd, *J* = 4.7 and 2.3 Hz, 1 H), 3.20 (m, 1 H), 2.92 (dd, *J* = 4.9 and 2.3 Hz, 1 H), 1.22 (d, *J* = 7.3 Hz, 3 H), 1.19 (d, *J* = 6.7 Hz, 3 H), 0.87 (s, 9 H), 0.07 (s, 3 H), and 0.06 (s, 3 H).

¹³C NMR (62.9 MHz; CDCl₃): δ 204.4, 168.3, 65.3, 61.7, 51.3, 47.6, 45.0, 25.7, 22.5, 17.9, 12.2, - 4.3, and - 5.0.

### EXAMPLE 5

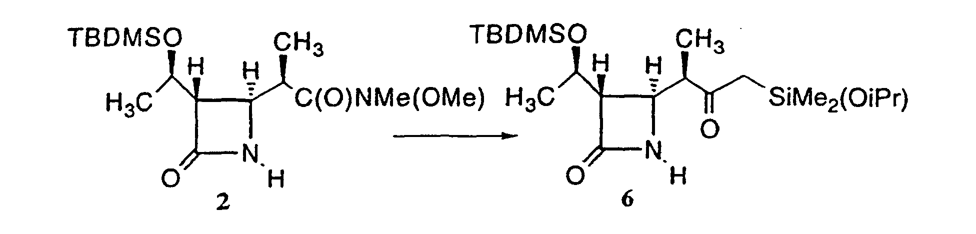

To a solution of **2** (52 mg) in dry tetrahydrofuran (2 mL) was added a solution of (isopropyloxydimethylsilyl)methylmagnesium chloride (0.7 *M*; 1.1 mL) in diethyl ether under ice-cooling. After the mixture was stirred under the ice bath for 2 hours, additional (isopropyloxydimethylsilyl)methylmagnesium chloride (0.7 *M*; 0.5 mL) in diethyl ether was added. After additional 1 hour under the ice bath, to the mixture was added saturated aqueous ammonium chloride (5 mL). The mixture was extracted with ethyl acetate (20 mL), dried over magnesium sulfate, purified by a silica gel column chromatography to give **6** (41 mg).

¹H NMR (250 MHz; CDCl₃): δ 6.02 (broad s, 1 H), 4.17 (m, 1 H), 4.01 (q, *J* = 6.0 Hz, 1 H), 3.86 (dd, *J* = 4.4 and 2.2 Hz, 1 H), 2.88 (dd, *J* = 4.2 and 2.2 Hz, 1 H), 2.83 (m, 1 H), 2.38 (d, *J* = 10.8 Hz, 1 H), 2.25 (d, *J* = 10.8 Hz, 1 H), 1.17 (d, *J* = 6.4 Hz, 3 H), 1.134 (d, *J* = 6.0 Hz, 6 H), 1.125 (d, *J* = 7.1 Hz, 3 H), 0.85 (s, 9 H), 0.20 (s, 6 H), and 0.04 (s, 6 H).

¹³C NMR (62.9 MHz; CDCl₃): δ 211.0, 168.4, 65.7, 65.2, 61.3, 50.9, 49.0, 38.5, 25.7, 25.6, 22.5, 17.9, 11.4, -0.66, -0.70, - 4.3, and -5.0.

## Claims

1. A process of synthesizing a compound of formula 1: 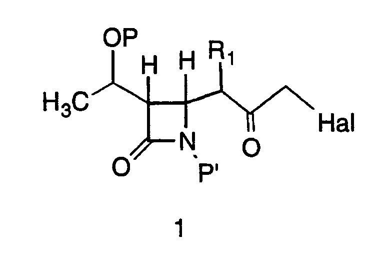 wherein P and P' each independently represent H or a protecting group, R¹ represents H or C₁₋₄ alkyl, and
Hal represents a halogen selected from Cl, Br and I, comprising:
reacting a compound of formula 2: 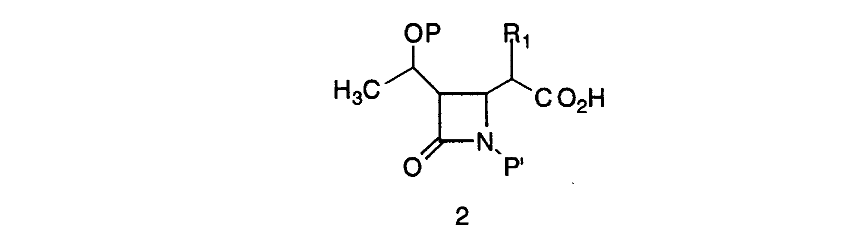 wherein P, P' and R₁ are as defined above with a di-C₁₋₄ alkyl hydroxylamine in the presence of a carbodiimide to produce a compound of formula 3: 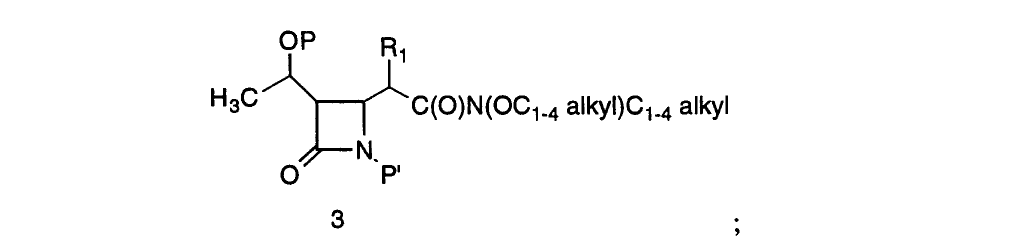
reacting compound 3 with a compound of formula 4:
MetCH₂SiR₂R₃R₄ 4
wherein Met represents lithium or halomagnesium;
R₂, R₃ and R₄ are C₁₋₄ alkyl or C₁₋₄ alkoxy, and the halo portion of halomagnesium is Cl, Br or I,
to produce a compound of formula 5: 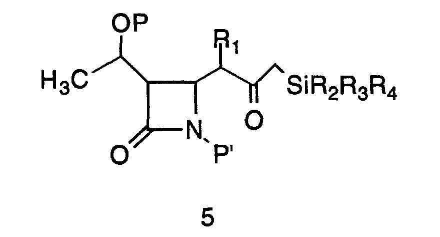 and
reacting compound 5 with a halogenating agent to produce a compound of formula 1.

2. A process in accordance with claim 1 wherein R₂ and R₃ are selected from methyl, ethyl, n-propyl, isopropyl, n-butyl and t-butyl, and R₄ represents C₁₋₄ alkoxy.

3. A process in accordance with claim 1 wherein R4 represents isopropoxy.

4. A process in accordance with claim 1 wherein P and P' represent H.

5. A process in accordance with claim 1 wherein P represents a protecting group and P' represents H.

6. A process in accordance with claim 5 wherein P is selected from the group consisting of: t-butylmethylphenylsilyl, t-butyldiphenylsilyl, trimethylsilyl, triethylsilyl, t-butyldimethylsilyl, o-nitrobenzyloxycarbonyl, p-nitrobenzyloxycarbonyl, p-nitrobenzyl, benzyloxycarbonyl, t-butyloxycarbonyl, 2,2,2-trichloroethyloxycarbonyl and allyloxycarbonyl.

7. A process in accordance with claim 6 wherein P represents a member selected from t-butyldimethylsilyl, trimethylsilyl and triethylsilyl.

8. A process in accordance with claim 1 wherein R¹ represents C₁₋₄ alkyl.

9. A process in accordance with claim 8 wherein R¹ represents methyl.

10. A process in accordance with claim 1 wherein the di-C₁₋₄ alkyl hydroxylamine is N,O-dimethylhydroxyamine.

11. A process in accordance with claim 1 wherein the carbodiimide is selected from 1-(3-dimethylaminopropyl)-3-ethylcarbodiimide and 1,3-dicyclohexylcarbodiimide.

12. A process in accordance with claim 1 wherein compound 5 is reacted with a halogenating agent selected from Br₂, I₂ , Cl₂ , N-chlorosuccinimide, N-bromosuccinimide, ICl and SO₂Cl₂ to produce a compound of formula 1.

13. A process in accordance with claim 12 wherein the halogenating compound is Br₂.

14. A process in accordance with claim 12 wherein the halogenating agent is SO₂Cl₂.

15. A process according to claim 1
wherein the di-C₁₋₄ alkylhydroxylamine is N, O-dimethylhydroxylamine, the carbodiimide is 1-(3-dimethylaminopropyl)-3-ethylcarbodiimide or 1,3-dicyclohexylcarbodiimide and the halogenating agent is selected from Br₂, I₂ , Cl₂ ,ICl, SO₂Cl₂ , NCS and NBS.

16. A process of synthesizing a compound of formula 1': 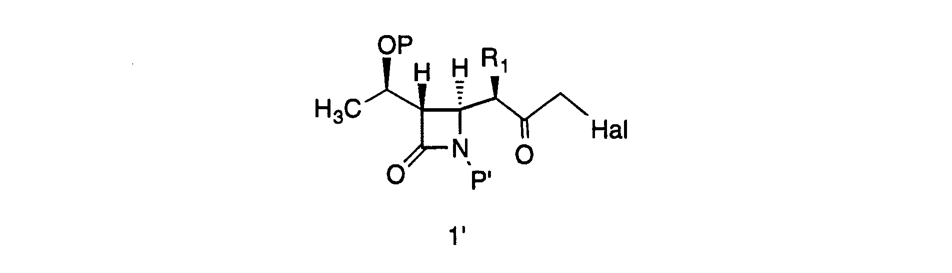 wherein P and P' each independently represent H or a protecting group, R¹ represents H or C₁₋₄ alkyl, and Hal represents a halogen selected from Cl, Br and I,
comprising:
reacting a compound of formula 2': 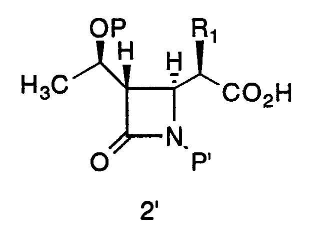 wherein P, P' and R₁ are as defined above with N, O-dimethylhydroxylamine in the presence of 1-(3-dimethylaminopropyl)-3-ethylcarbodiimide or 1,3-dicyclohexylcarbodiimide to produce a compound of formula 3': 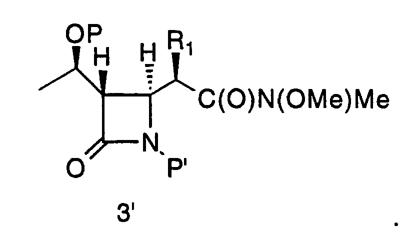
reacting a compound of formula 3' with a silyl compound of formula 4:
MetCH₂SiR₂R₃R₄ 4
wherein Met represents lithium or halomagnesium,
the halo portion of halomagnesium is Cl, Br or I, and R₂, R₃ and R₄ are independently C₁₋₄ alkyl or C₁₋₄ alkoxy, to produce a compound of formula 5': 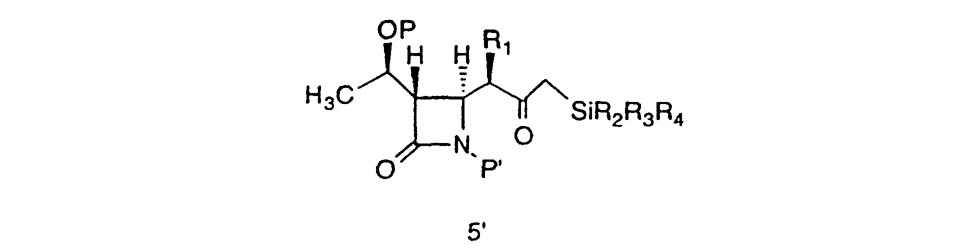 and reacting compound 5' with a halogenating agent selected from Br₂, I₂, Cl₂ , NBS, NCS, ICl and SO₂Cl₂ to produce a compound of formula 1'.

17. A compound represented by formula 3 or 5: 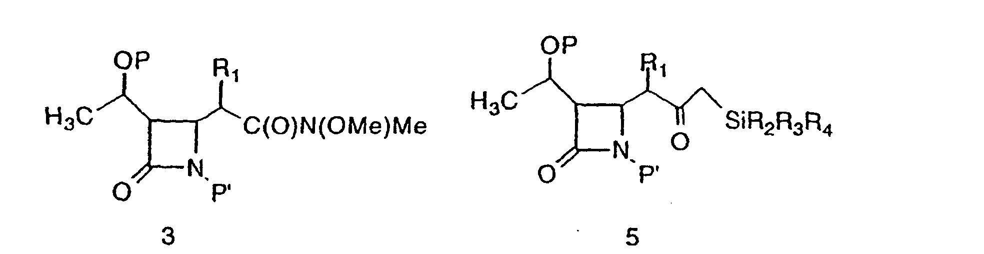 wherein
R₁ represents H or C₁₋₄ alkyl,
P and P' independently represent H or a protecting group selected from the group consisting of: t-butylmethylphenylsilyl, t-butyldiphenylsilyl, trimethylsilyl, triethylsilyl, t-butyldimethylsilyl, o-nitrobenzyloxycarbonyl, p-nitrobenzyloxycarbonyl, p-nitrobenzyl, benzyloxycarbonyl, t-butyloxycarbonyl, 2,2,2-trichloroethyloxycarbonyl and allyloxycarbonyl and R₂, R₃ and R₄ are C₁₋₄ alkyl or C₁₋₄ alkoxy.

18. A compound according to claim 17 of formula 3 in which P is t-butyldimethylsilyl, R¹ is CH₃ and P' is H.

## Patentansprüche

1. Ein Verfahren zur Synthese einer Verbindung der Formel 1: 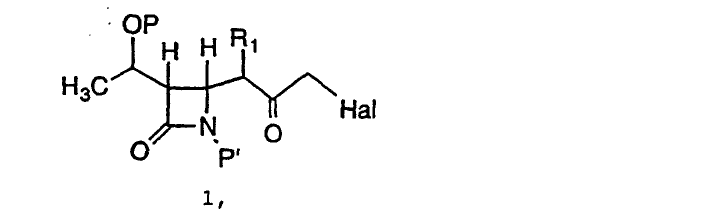 wobei P und P' jeweils unabhängig H oder eine Schutzgruppe bedeuten,
R¹ H oder C₁₋₄-Alkyl bedeutet und
Hal ein Halogen bedeutet, ausgewählt aus Cl, Br und I, umfassend:
Umsetzung einer Verbindung der Formel 2: 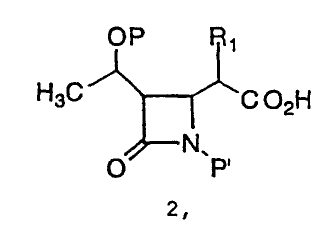 wobei P, P' und R₁ wie oben definiert sind, mit einem Di-C₁₋₄-alkylhydroxylamin in Gegenwart eines Carbodiimids, um eine Verbindung der Formel 3 zu erzeugen: 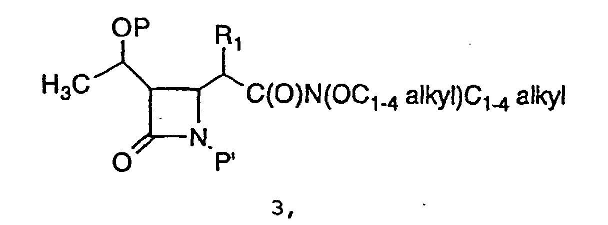
Umsetzung von Verbindung 3 mit einer Verbindung der Formel 4:
MetCH₂SiR₂R₃R₄ 4,
wobei Met Lithium oder Halogenmagnesium bedeutet,
R₂, R₃ und R₄ C₁₋₄-Alkyl oder C₁₋₄-Alkoxy sind und der Halogenteil von Halogenmagnesium Cl, Br oder I ist,
um eine Verbindung der Formel 5 zu erzeugen: 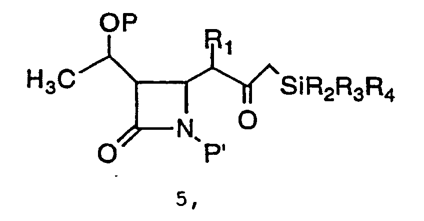 und
Umsetzung von Verbindung 5 mit einem Halogenierungsmittel, um eine Verbindung der Formel 1 zu erzeugen.

2. Ein Verfahren gemäß Anspruch 1, wobei R₂ und R₃ aus Methyl, Ethyl, n-Propyl, Isopropyl, n-Butyl und t-Butyl ausgewählt sind und R₄ C₁₋₄-Alkoxy bedeutet.

3. Ein Verfahren gemäß Anspruch 1, wobei R₄ Isopropoxy bedeutet.

4. Ein Verfahren gemäß Anspruch 1, wobei P und P' H bedeuten.

5. Ein Verfahren gemäß Anspruch 1, wobei P eine Schutzgruppe bedeutet und P' H bedeutet.

6. Ein Verfahren gemäß Anspruch 5, wobei P ausgewählt ist aus der Gruppe, bestehend aus: t-Butylmethylphenylsilyl, t-Butyldiphenylsilyl, Trimethylsilyl, Triethylsilyl, t-Butyldimethylsilyl, o-Nitrobenzyloxycarbonyl, p-Nitrobenzyloxycarbonyl, p-Nitrobenzyl, Benzyloxycarbonyl, t-Butyloxycarbonyl, 2,2,2-Trichlorethyloxycarbonyl und Allyloxycarbonyl.

7. Ein Verfahren gemäß Anspruch 6, wobei P ein Element bedeutet, ausgewählt aus t-Butyldimethylsilyl, Trimethylsilyl und Triethylsilyl.

8. Ein Verfahren gemäß Anspruch 1, wobei R¹ C₁₋₄-Alkyl bedeutet.

9. Ein Verfahren gemäß Anspruch 8, wobei R¹ Methyl bedeutet.

10. Ein Verfahren gemäß Anspruch 1, wobei das Di-C₁₋₄-alkylhydroxylamin N,O-Dimethylhydroxylamin ist.

11. Ein Verfahren gemäß Anspruch 1, wobei das Carbodiimid ausgewählt ist aus 1-(3-Dimethylaminopropyl)-3-ethylcarbodiimid und 1,3-Dicyclohexylcarbodiimid.

12. Ein Verfahren gemäß Anspruch 1, wobei Verbindung 5 mit einem Halogenierungsmittel, ausgewählt aus Br₂, I₂, Cl₂, N-Chlorsuccinimid, N-Bromsuccinimid, ICl und SO₂Cl₂, umgesetzt wird, um eine Verbindung der Formel 1 zu erzeugen.

13. Ein Verfahren gemäß Anspruch 12, wobei die Halogenierungsverbindung Br₂ ist.

14. Ein Verfahren gemäß Anspruch 12, wobei das Halogenierungsmittel SO₂Cl₂ ist.

15. Ein Verfahren gemäß Anspruch 1, wobei das Di-C₁₋₄-alkylhydroxylamin N,O-Dimethylhydroxylamin ist, das Carbodiimid 1-(3-Dimethylaminopropyl)-3-ethylcarbodiimid oder 1,3-Dicyclohexylcarbodiimid ist und das Halogenierungsmittel ausgewählt ist aus Br₂, I₂, Cl₂, ICl, SO₂Cl₂, NCS und NBS.

16. Ein Verfahren zur Synthese einer Verbindung der Formel 1': 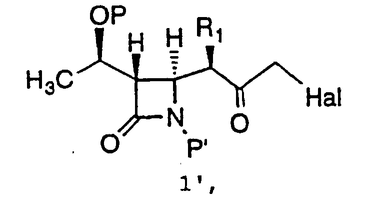 wobei P und P' jeweils unabhängig H oder eine Schutzgruppe bedeuten, R¹ H oder C₁₋₄-Alkyl bedeutet und Hal ein Halogen bedeutet, ausgewählt aus Cl, Br und I, umfassend:
Umsetzung einer Verbindung der Formel 2': 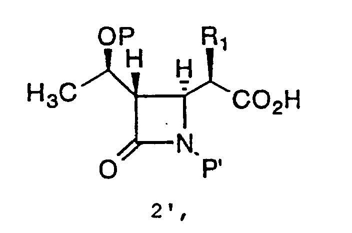 wobei P, P' und R₁ wie oben definiert sind, mit N,O-Dimethylhydroxylamin in Gegenwart von 1-(3-Dimethylaminopropyl)-3-ethylcarbodiimid oder 1,3-Dicyclohexylcarbodiimid, um eine Verbindung der Formel 3' zu erzeugen: 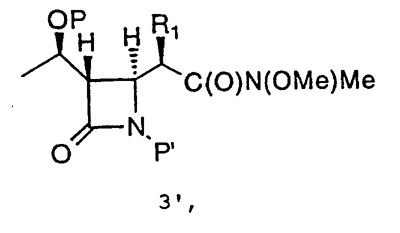
Umsetzung einer Verbindung der Formel 3' mit einer Silylverbindung der Formel 4:
MetCH₂SiR₂R₃R₄ 4,
wobei Met Lithium oder Halogenmagnesium bedeutet,
der Halogenteil von Halogenmagnesium Cl, Br oder I ist und R₂, R₃ und R₄ unabhängig C₁₋₄-Alkyl oder C₁₋₄-Alkoxy sind, um eine Verbindung der Formel 5' zu erzeugen: 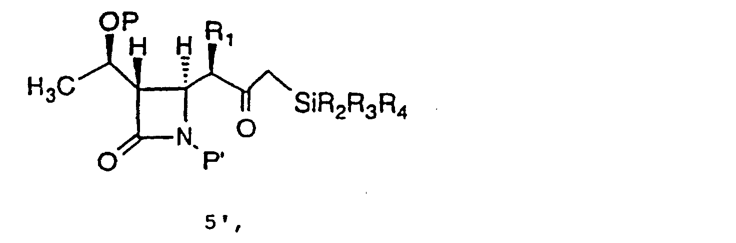 und Umsetzung von Verbindung 5' mit einem Halogenierungsmittel, ausgewählt aus Br₂, I₂, Cl₂, NBS, NCS, ICl und SO₂Cl₂, um eine Verbindung der Formel 1' zu erzeugen.

17. Eine Verbindung, dargestellt durch Formel 3 oder 5: 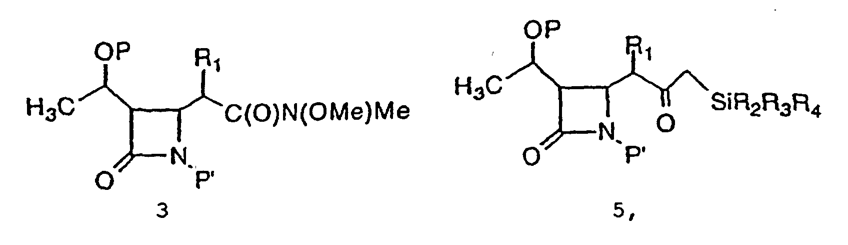 wobei
R₁ H oder C₁₋₄-Alkyl bedeutet,
P und P' unabhängig H oder eine Schutzgruppe, ausgewählt aus der Gruppe, bestehend aus: t-Butylmethylphenylsilyl, t-Butyldiphenylsilyl, Trimethylsilyl, Triethylsilyl, t-Butyldimethylsilyl, o-Nitrobenzyloxycarbonyl, p-Nitrobenzyloxycarbonyl, p-Nitrobenzyl, Benzyloxycarbonyl, t-Butyloxycarbonyl, 2,2,2-Trichlorethyloxycarbonyl und Allyloxycarbonyl, bedeuten und R₂, R₃ und R₄ C₁₋₄-Alkyl oder C₁₋₄-Alkoxy sind.

18. Eine Verbindung gemäß Anspruch 17 der Formel 3, wobei P t-Butyldimethylsilyl ist, R¹ CH₃ ist und P' H ist.

## Revendications

1. Procédé de synthèse d'un composé de formule 1 : 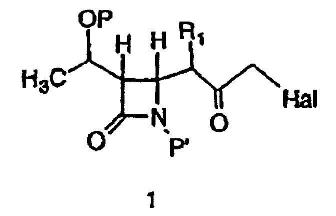 dans laquelle P et P' représentent chacun indépendamment H ou un groupe protecteur,
R¹ représente H ou un groupe alkyle en C₁₋₄, et
Hal représente un atome d'halogène choisi parmi Cl, Br et I,
comprenant le fait de :
faire réagir un composé de formule 2 : 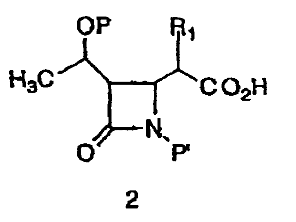 dans laquelle, P, P' et R₁ sont tels que définis ci-dessus, avec une di-(alkyle en C₁₋₄)hydroxylamine en présence d'un carbodiimide pour produire un composé de formule 3 : 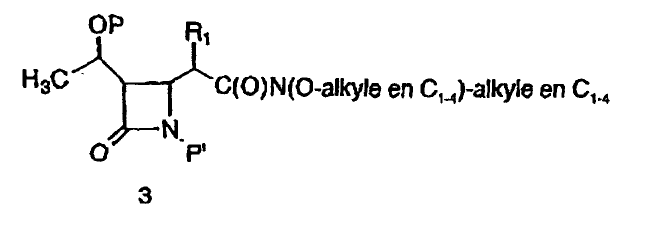
faire réagir le composé 3 avec un composé de formule 4 :
MétCH₂SiR₂R₃R₄ 4
dans laquelle Mét représente le lithium ou un halogénomagnésium ;
R₂, R₃ et R₄ représentent un groupe alkyle en C₁₋₄ ou alcoxy en C₁₋₄, et la partie halogène de l'halogénomagnésium est Cl, Br ou I,
pour produire un composé de formule 5 : 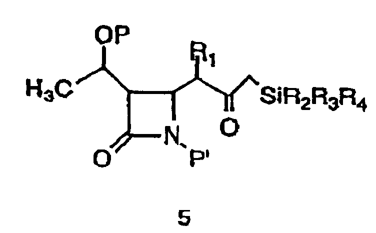 et
faire réagir un composé 5 avec un agent halogénant pour produire un composé de formule 1.

2. Procédé selon la revendication 1, dans lequel R₂ et R₃ sont choisis parmi les groupes méthyle, éthyle, n-propyle, isopropyle, n-butyle et t-butyle, et R₄ représente un groupe alcoxy en C₁₋₄.

3. Procédé selon la revendication 1, dans lequel R₄ représente un groupe isopropoxy.

4. Procédé selon la revendication 1, dans lequel P et P' représentent H.

5. Procédé selon la revendication 1, dans lequel P représente un groupe protecteur et P' représente H.

6. Procédé selon la revendication 5, dans lequel P est choisi dans l'ensemble consistant en groupes t-butylméthylphénylsilyle, t-butyldiphénylsilyle, triméthylsilyle, triéthylsilyle, t-butyldiméthylsilyle, o-nitrobenzyloxycarbonyle, p-nitrobenzyloxycarbonyle, p-nitrobenzyle, benzyloxycarbonyle, t-butyloxycarbonyle, 2,2,2-trichloroéthyloxycarbonyle et allyloxycarbonyle.

7. Procédé selon la revendication 6, dans lequel P représente un membre choisi parmi les groupes t-butyldiméthylsilyle, triméthylsilyle et triéthylsilyle.

8. Procédé selon la revendication 1, dans lequel R¹ représente un groupe alkyle en C₁₋₄.

9. Procédé selon la revendication 8, dans lequel R¹ représente un groupe méthyle.

10. Procédé selon la revendication 1, dans lequel la di-(alkyle en C₁₋₄)hydroxylamine est la N,O-diméthylhydroxyamine.

11. Procédé selon la revendication 1, dans lequel le carbodiimide est choisi parmi le 1-(3-diméthylaminopropyl)-3-éthylcarbodiimide et le 1,3-dicyclohexylcarbodiimide.

12. Procédé selon la revendication 1, dans lequel on fait réagir le composé 5 avec un agent halogénant choisi parmi Br₂, I₂, Cl₂, le N-chlorosuccinimide, le N-bromosuccinimide, ICl et SO₂Cl₂ pour produire un composé de formule 1.

13. Procédé selon la revendication 12, dans lequel le composé halogénant est Br₂.

14. Procédé selon la revendication 12, dans lequel l'agent halogénant est SO₂Cl₂.

15. Procédé selon la revendication 1, dans lequel la di-(alkyle en C₁₋₄)hydroxylamine est la N,O-diméthylhydroxylamine, le carbodiimide est le 1-(3-diméthylaminopropyl)-3-éthylcarbodiimide ou le 1,3-dicyclohexylcarbodiimide, et l'agent halogénant est choisi parmi Br₂, I₂, Cl₂, ICl, SO₂Cl₂, NCS et NBS.

16. Procédé de synthèse d'un composé de formule 1' 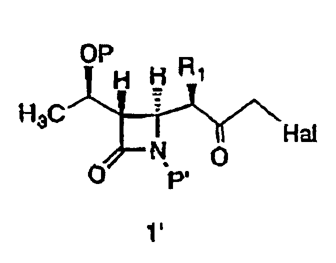 dans lequel P et P' représentent chacun indépendamment H ou un groupe protecteur,
R¹ représente H ou un groupe alkyle en C₁₋₄, et
Hal représente un atome d'halogène choisi parmi Cl, Br et I,
comprenant le fait de :
faire réagir un composé de formule 2' : 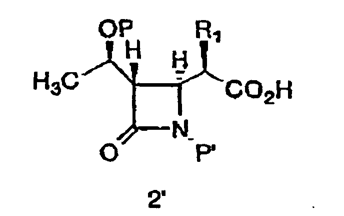 dans laquelle P, P' et R₁ sont tels que définis ci-dessus avec de la N,O-diméthylhydroxylamine en présence de 1-(3-diméthylaminopropyl)-3-éthylcarbodiimide ou de 1,3-dicyclohexylcarbodiimide pour produire un composé de formule 3' : 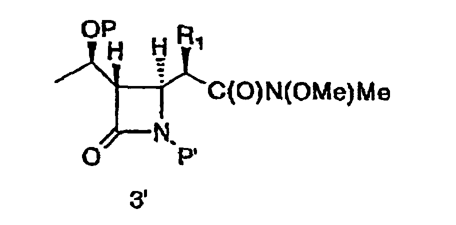
faire un réagir un composé de formule 3' avec un composé silyle de formule 4 :
MétCH₂SiR₂R₃R₄ 4
dans laquelle Mét représente le lithium ou un halogénomagnésium,
la partie halogéno de l'halogénomagnésium est Cl, Br ou I, et
R₂, R₃ et R₄ représentent indépendamment un groupe alkyle en C₁₋₄ ou un groupe alcoxy en C₁₋₄, pour produire un composé de formule 5' : 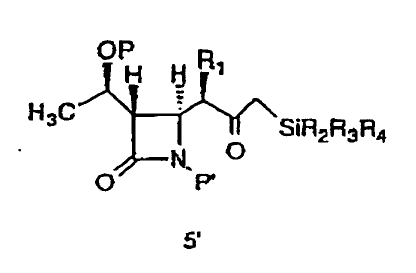 et faire réagir le composé 5' avec un agent halogénant choisi parmi Br₂, I₂, Cl₂, NBS, NCS, ICl et SO₂Cl₂ pour produire un composé de formule 1'.

17. Composé représenté par la formule 3 ou 5 : 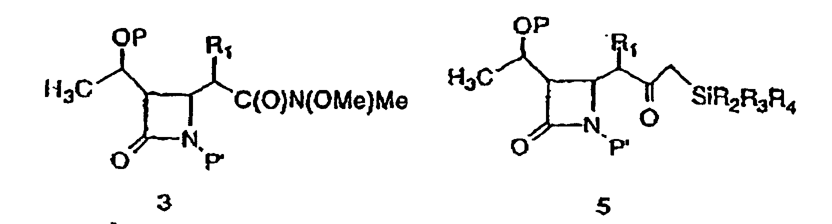 dans lesquelles
R₁ représente H ou un groupe alkyle en C₁-C₄, P et P' représentent indépendamment H ou un groupe protecteur choisi dans l'ensemble consistant en groupes t-butylméthylphénylsilyle, t-butyldiphénylsilyle, triméthylsilyle, triéthylsilyle, t-butyldiméthylailyle, o-nitrobenzyloxycarbonyle, p-nitrobenzyloxycarbonyle, p-nitrobenzyle, benzyloxycarbonyle, t-butyloxycarbonyle, 2,2,2-trichloroéthyloxycarbonyle et allyloxycarbonyle,
et R₂, R₃ et R₄ représentent un groupe alkyle en C₁₋₄ ou un groupe alcoxy en C₁₋₄.

18. Composé selon la revendication 17 de formule 3, dans laquelle P représente un groupe t-butyldiméthylsilyle, R¹ représente CH₃ et P' représente H.
